# EUROPÄISCHE PATENTSCHRIFT

(11) **EP 1 032 379 B1**
(45) Veröffentlichungstag und Bekanntmachung des Hinweises auf die Patenterteilung: **12.12.2001**
(21) Anmeldenummer: 98962351.7
(22) Anmeldetag: 18.11.1998
(51) Int. Cl.: A61K 31/20, A61K 47/00, A61K 47/24

(54) **ZUSAMMENSETZUNG MIT AZELAINSÄURE**
COMPOSITION WITH AZELAIC ACID
COMPOSITION COMPRENANT DE L'ACIDE AZELAIQUE

(30) Priorität: 19.11.1997 DE 19753044; 12.02.1998 US 74850 P; 20.02.1998 DE 19808086
(43) Veröffentlichungstag der Anmeldung: 06.09.2000
(73) Patentinhaber: SCHERING AKTIENGESELLSCHAFT, 13353 Berlin (DE)
(72) Erfinder: FRANKE, Patrick, D-10711 Berlin (DE); GÜNTHER, Clemens, D-13357 Berlin (DE); RIEDL, Jutta, D-79594 Inzlingen (DE)
(74) Vertreter: Dörries, Hans Ulrich, Dr.
(86) Internationale Anmeldenummer: EP9807370
(87) Internationale Veröffentlichungsnummer: WO9925332

(56) Entgegenhaltungen:
- WO-A-95/04537
- WO-A-95/05163
- WO-A-96/05806
- WO-A-96/39119

## Beschreibung

Die Erfindung, betrifft eine Zusammensetzung mit Azelainsäure in einem Hydrogel die als Arzneimitte verwendet werden Kann. Diese Anmeldung nimmt die deutschen Prioritäten DE 197 53 044 mit dem Anmeldetag vom 19. November 1997 und DE 198 08 086 mit dem Anmeldetag vom 20. Februar 1998 in Anspruch. Für die USA wird die US - Hinterlegung US-SN: 60/074.850 mit dem Einreichungsdatum vom 12 Februar 1998 ais zusätzliche Priorität beansprucht.

### Stand der Technik

In der EP 0 336 880 A2. die am 29.3.1998 angemeldet worden ist, wird eine pharmazeutische Zusammensetzung beschrieben, die besteht aus (i) Azelainsäure in einer Konzentration von 20 Gewichtsprozenten, aus (iii) Triacylglyceriden und Diacylglyceriden, aus (iv) Propylenglycol. aus (v) Polysorbat, zum Beispiel Polyethylen(20)sorbitanmonooleat, und aus (vii) Wasser und Salzen, Diese topisch zu verabreichende Zusammensetzung wird zur Behandlung von verschiedenen altersbedingten Veränderungen der Gesichtshaut eingesetzt. Die Zusammensetzung liegt als Creme vor. Bekannt ist weiterhin, die Azelainsäure auch gegen entzündliche und infektiöse Dermatosen, wie zum Beispiel Akne und Rosazea. einzusetzen.

In der Roten Liste von 1996 (ISBN 3 - 87193 - 167 -5) wird unter der Nummer 32 282 eine pharmazeutische Zusammensetzung mit dem Namen Skinoren® beschrieben, die besteht aus (i) Azelainsäure in einer Konzentration von 20 Gewichtsprozenten, aus (iii) Triacylglyceriden und Diacylglyceriden aus (iv) Propylenglycol, aus (v) Polysorbat, zum Beispiel Macrogolstrearat 1000 und aus (vii) Wasser und Salzen. Diese topisch zu verabreichende Zusammensetzung wird zur Behandlung von Akne eingesetzt. Die Zusammensetzung liegt als Creme vor. Dieses Dokument wird als nächster Stand der Technik angesehen.

Die internationale Anmeldung WO 96/11700, die am 29. Oktober 1993 hinterlegt worden ist. beschreibt eine pharmazeutische Zusammensetzung. die als Hilfsstoff für einen lmpfstoff eingesetzt wird. Diese Zusammensetzung soll das Freund'sche Adjuvanz ersetzen. Sie wird injiziert. Als (i) pharmazeutischer Wirkstoff wird zum Beispiel Hepatitis B Oberflächen - Proteine eingesetzt. Weiterhin werden (ii) Polyacrylsäure, (iii) Triacylglyceride und / oder Diacylglyceride, wie MIGLYOL, (iv) Propylenglycol, und (v) Polysorbate in Form von TWEEN. EMULROR und SIMULSOL M-53 verwendet. Ebenfalls wird (vi) Sojalecithin hinzugegeben. Die Zusammensetzung ist eine (vii) wäßrige Phase mit Salzen. Die Zusammensetzung wird nicht topisch verabreicht. Die Emulsion weist Partikel in der Größe von 0,03 bis 0,5 µm auf, bevorzugt 0.05 bis 0,2 µm.

Die internationale Anmeldung WO 95/05163. die am 5. August 1994 hinterlegt worden ist, beschreibt eine pharmazeutische Zusammensetzung, die als Emulsion zur Verabreichung von biologisch aktiven Substanzen auf der Hautoberfläche vorliegt. Diese Zusammensetzung enthält Partikel von einer Größe von 30 bis 500 nm. bevorzugt 70 bis 200 nm. Als (i) pharmazeutische Wirkstoffe werden zum Beispiel anti - inflamatorische Medikamente eingesetzt. Weiterhin werden (ii) Polyacrylsäure, (iii) Triacylglyceride und / oder Diacylgiyceride. (iv) Propylenglycol, und (v) Polysorbate in Form von TWEEN. EMULROR, TRITON X und SIMULSOL M-53 verwendet. Ebenfalls wird (vi) Sojalecithin hinzugegeben. Die Zusammensetzung ist eine (vii) wäßrige Phase mit Salzen. Die Zusammensetzung wird topisch verabreicht.

Die europäische Patentanmeldung EP 0 696 452, die am 26. Juli 1995 hinterlegt worden ist, beschreibt eine Nanoemulsion, die als Medikament zur Behandlung der Augen eingesetzt wird, wobei die Nanoemulsion als topisch aufzutragende Augentropfen verabreicht wird. Diese Zusammensetzung enthält Partikel von einer mittleren Größe von 520 nm. Als (i) pharmazeutische Wirkstoffe werden zum Beispiel anti - inflamatorische Medikamente eingesetzt. Weiterhin werden (ii) Polyacrylsäure, (iii) Triacylglyceride und / oder Diacylglyceride, (iv) Propylenglycol, und (v) Polysorbate in Form von Polyoxyethylenpolyoxypropylen Copolymere verwendet. Die Zusammensetzung ist eine (vii) wäßrige Phase mit Salzen. Die Zusammensetzung wird topisch verabreicht.

### Aufgabe und Lösung

Es stellt sich die Aufgabe, eine pharmazeutische Zusammensetzung mit Azelainsäure als therapeutischen Wirkstoff anzubieten. wobei die Bioverfügbarkeit der Azelainsäure gesteigert sein soll.

Bei einer Zusammensetzung gemäß dem Stand der Technik die folgenden Merkmale umfassend:
(i) Azefainsäure als therapeutische Wirkstoff,
(iii) Triacylglyceride,
(iv) Propylenglycol, und
(v) Polysorbate
(vii) in einer wäßrigen Phase, umfassend Wasser und Salze,
wird die Aufgabe dadurch gelöst,
daß die Zusammensetzung als weitere Zusätze
(ii) Polyacrytsäure und
(vi) Lecithin umfaßt, wobei das Lecithin in höchstens 3 Gewichtsprozent in der Zusammensehung vorliegt
und die Zusammensetzung ein Hydrogel ist.

### Vorteile

Die Zusammensetzung gemäß der Erfindung weist den Vorteil auf, daß sie eine höhere Menge an pharmazeutischern Wirkstoff in lebende Hautschichten und / oder Hautanhangsorganen gelangen läßt. Hierbei liegt die Verfügbarkeit der Azelainsäure bei der erfindungsgemäßen Zusammensetzung um die Faktoren drei bis fünf höher als bei der Azelainsäure - Creme gemäß dem Stand der Technik. Diese Verfügbarkeit wurde in einem FRANZ - Durchfluß-Diffusionszell - Test nachgewiesen, der in den Beispielen ausführlich dargestellt wird. Gerade die lebenden Hautschichten und / oder Hautanhangsorgane sind der gewünschten Zielorte für Azelainsäure. Besonders gut ist die Zusammensetzung bei Azelainsäure in hohen Konzentrationen einzusetzen.

### Weitere Ausgestaltung der Zusammensetzung

Vorteilhaft ist eine Zusammensetzung, welche topisch verabreichbar ist.

Bevorzugt ist eine erfindungsgemäße Zusammensetzung, bei der die Azelainsäure in einer Konzentration von 5 bis 20 Gewichtsprozent, mehr bevorzugt in einer Konzentration von 10 bis 18 Gewichtsprozent und am meisten bevorzugt in einer Konzentration von 14 bis 16 Gewichtsprozent vorliegt.

Wesentlich ist die Gegenwart von Polyacrylsäure. Sie ist für das Entstehen des Hydrogels entscheidend. In dem Gel ist als Lecithin das Sojalecithin bevorzugt. Das Lecithin oder Sojalecithin ist in einer Konzentration von höchstens 3 Gewichtsprozenten vorteilhaft. Mehr bevorzugt eine Konzentration von höchstens 1,5 Gewichtsprozenten und am meisten bevorzugt eine Konzentration von höchstens 1 Gewichtsprozent. Die letzte Konzentration bewirkt, daß das Hydrogel nicht mehr als Nanoemulsion vorliegt.

### Vorteile

Unerwartet hat sich herausgestellt, daß die erfindungsgemäße Zusammensetzung bei Konzentrationen an Lecithin von einem Gewichtsprozent und weniger keine klassische Nanoemulsion gemäß dem Stand der Technik bildet. Vielmehr liegt ein Gel vor, welches eine homogene Masse mit praktisch keinen Vesikeln, jedoch Membranfragmenten umfaßt. Daß Azelainsäure und der Rest der Zusammensetzung keine Nanoemulsion bilden, war nicht vorauszusehen. Erst mit Hilfe von Raster ― Elektronenmikroskop - Aufnahmen konnte Klarheit geschaffen werden. Es stellte sich heraus, daß keine Nanoemulsion bei mikroskopischer Betrachtung zu identifizieren ist. Vorteilhafter Weise besitzt die erfindungsgemäße Zusammensetzung eine hohe Penetration in lebende Hautschichten und / oder Hautanhangsorganen, die bei Cremen nicht zu beobachten ist

### Bevorzugte Ausführungsformen

Bevorzugt ist eine Zusammensetzung, bei der die einzelnen Parameter unabhängig voneinander die folgenden Konzentrationen aulweisen können:
(ii) Polyacrylsäure in einer Konzentration von 0.5 bis 2 Gewichtsprozenten,
(iii) Triacylglyceride in einer Konzentration von 0,5 bis 5 Gewichtsprozenten,
(iv) Propylenglycol in einer Konzentration von 5 bis 15 Gewichtsprozenten und
(v) Polysorbate in einer Konzentration von 0,5 bis 3 Gewichtsprozenten.

Die Bestandteile sind selbstverständlich so aufeinander abzustimmen, daß eine Summe von 100% erzielt wird.

Am meisten bevorzugt ist eine Zusammensetzung, bei der die einzelnen Parameter unabhängig voneinander die folgenden Konzentrationen aufweisen können:
(ii) Polyacrylsäure in einer Konzentration von 1 = 0.25 Gewichtsprozenten,
(iii) Triacylglyceride in einer Konzentration von 2 ± 1 Gewichtsprozenten,
(iv) Propylenglycol in einer Konzentration von 10 ± 2 Gewichtsprozenten und
(v) Polysorbate in einer Konzentration von 2 ± 0,5 Gewichtsprozenten.

Die Bestandteile sind selbstverständlich so aufeinander abzustimmen, daß eine Summe von 100% erzielt wird.

### Definitionen

Azelainsäure und deren Herstellung ist in dem deutschen Patent DE 28 17 133.7 beschrieben. Vergleiche auch Römpp, Chemie Lexikon. Herausgeb. Jürgen FALBE und Manfred REGNITZ. 1989, 9. Auflage. Seite 323. Thieme Verlag Stuttgart, ISBN 3-13-734609-6.

Polyacrylsäure stellt ein anionenaktives Polymerisat aus Akrylsäure dar, das in Wasser nur zum Teil löslich ist. Die einprozentige wäßrige Suspension besitzt einen pH-Wert von 3 und annähernd gleiche Viskosität wie Wasser Erst beim Neutralisieren mit anorganischen oder organischen Basen kommt es zur Gelbildung und zum Entstehen hochviskoser Produkte. Rudolf VOIGT und Manfred BORNSCHEIN, 1979, Lehrbuch der pharmazeutischen Technologie, Seite 314. VEB Verlag Volk und Gesundheit Berlin. Vergleiche auch Römpp. Chemie Lexikon. Herausgeb. Jürgen FALBE und Manfred REGNITZ. 1992, 9. Auflage. Seite 3508. Thieme Verlag Stuttgart, ISBN 3-13-735009-3.

"Triglycerid" ist eine Bezeichnung für Ester des Glycerins, dessen drei Hydroxy-Gruppen durch Karbonsäuren verestert sind. Die natürlich vorkommenden Fette und fetten Öle sind Triglyceride ("Neutralfette"), die in der Regel verschiedene Fettsäuren im gleichen Glycerin-Molekül enthalten. J. Am.- Oil. Chem Soc. Vol 62, Seite 730, (1985); und Parfüm. Kosmet. Vol 58, Seite 353, (1977); und Römpp, Chemie Lexikon, Herausgeb. Jürgen FALBE und Manfred REGNITZ. 1990. 9. Auflage. Seiten 1339 - 1342. Thieme Verlag Stuttgart, ISBN 3-13-734709-2.

Propylenglycol ist in in H.P. FIEDLER: Lexikon der Hilfsstoffe. 4. Auflage. 1996, ISBN 3-87193-173 auf den Seiten 1278 bis 1282 beschrieben.

Polysorbate sind in H.P. FIEDLER: Lexikon der Hilfsstoffe, 4. Auflage. 1996. ISBN 3-87193-173 auf den Seiten 1251 bis 1252 beschrieben.

Lecithine werden durch Extrahieren aus biologischem Material gewonnen. So umfaßt eine Lecithin - Fraktion aus Sojabohnen (dem gebräuchlichsten Rohstoff) z.B. Palmitinsäure, Stearinsäure, Palmitoleinsäure, Oleinsäure, Linolsäure u. Linolensäure. Normalerweise ist die gesättigte Fettsäure mit der primären, die ungesättigte mit der sekundären Hydroxy-Gruppe des Glyzerins verestert. Lecithine sind Bestandteile der Zellmembranen aller Lebewesen. In Wasser quellen Lecithine zunächst wie lyophile Kolloide auf. Später stellen sie durchsichtige, kolloidale Lösungen dar. Je nach Wassergehalt bilden sie unterschiedlichen Texturen, wobei die Lamellen aus Lipid - Doppelschichten gebildet werden. Bei höherem Wassergehalt entstehen Liposomen. Lit.: Pardun, Die Pflanzenlecithine, Augsburg: Verl. für Chem. Ind. (Ziolkowsky KG) 1988. Weitere Lecithine und deren Wirkung sind beschrieben in J. GAREISS et al. 1994. Pafümerie und Kosmetik, Vol. 10/94, Seiten 652 - 659. Hüthing GmbH Heidelberg.

Ein Gel zeichnet sich durch die folgenden Eigenschaften aus: Es handelt sich um ein formbeständiges, leicht deformierbares, an Flüssigkeit und gegebenenfalls Gasen reiches, disperses System aus mindestens zwei Komponenten. Römpp. Chemie Lexikon, Herausgeb. Jürgen FALBE und Manfred REGNITZ, 1990, 9. Auflage, Seite 1511, Thieme Verlag Stuttgart, ISBN 3-13-734709-2; und Pharm. Unserer Zeit Vol. 8, Seiten 179 bis 188. (1979): und Parfüm. Kosmet.. Vol. 58, Seiten 251 bis 253 (1977)

Konservierungsmittel können in der wäßrigen Phase enthalten sein. Zu den Konservierungsmittel gehört zum Beispiel Benzoesäure. Aufgrund ihrer antimikrobiellen Eigenschaft ist Benzoesäure als Konservierungsmittel besonders geeignet.

### Eigenschaften bei der Verwendung als Medikament

Die Zusammensetzung der Erfindung zeigt pharmakologische Wirkung und ist als therapeutischer Wirkstoff oder als Medikament einsetzbar.

Bevorzugt ist eine erfindungsgemäße Zusammensetzung zusammen mit mindestens einem physiologisch verträglichen, pharmakologischen Hilfsstoff oder Trägerstoff. Pharmakologische Hilfsstoffe und Trägerstoffe sind in Remington's Pharmaceutical Science, 15^{th} ed. Mack Publishing Company, Easton Pennsylvania (1980) beschrieben.

Die Zusammensetzung der Erfindung eignet sich zur Behandlung verschiedener Indikationen. Bevorzugt ist eine Zusammensetzung der Erfindung als therapeutischer Wirkstoff zur Behandlung von Rosacea, Altershaut. Melasma, Akne und / oder Hautirritationen. Mehr bevorzugt ist eine Zusammensetzung der Erfindung als therapeutischer Wirkstoff zur Behandlung von Rosacea. Altershaut. Melasma. Akne und / oder Hautirritationen zusammen mit mindestens einem physiologisch verträglichen, pharmakologischen Hilfsstoff oder Trägerstoff. Die Behandlung umfaßt prophylaktische und therapeutische Maßnahmen.
(i) Die Erfindung liefert weiterhin
   (α) die Verwendung der pharmazeutischen Zusammensetzung der Erfindung zur Herstellung eines Medikaments zur Behandlung von Rosacea, Altershaut, Melasma, Akne und / oder Hautirritationen;
   (β) eine pharmazeutische Zusammensetzung zur Behandlung von Rosacea, Altershaut, Melasma, Akne und / oder Hautirritationen. welche Behandlung eine pharmazeutische Zusammensetzung der Erfindung und wenigstens einen pharmazeutisch verträglichen Träger und Zusatz umfaßt.

Für diese therapeutische Wirkungen ist die geeignete Dosis unterschiedlich und hängt beispielsweise von der Konzentration der Einzelelemente in der pharmazeutischen Zusammensetzung ab, dem Wirt, der Art der Verabreichung und der Art und der Schwere der zu behandelnden Zustände ab

Die pharmazeutische Zusammensetzung der Erfindung kann mit den in der galenischen Pharmazie üblichen Zusätzen und/oder Trägersubstanzen nach an sich bekannten Methoden zu den üblichen topischen Applikationsformen verarbeitet werden.

### Beispiele

### 1. Herstellung der pharmazeutischen Zusammensetzung

Eine pharmazeutische Zusammensetzung, welche Azelainsäure enthält, weist die folgende Rezeptur und die folgenden Verfahrensschritte auf:

Benzoesäure und EDTA werden in üblichen Konzentrationen in 60 bis 70 Teilen Wasser gelöst. Anschließend wird eine Mischung von 1 Teil mittelkettigen Triglyceriden und 1,5 Teilen Polysorbat 80 unter Rühren hinzugegeben und homogenisiert (Voremulsion). 1 Teil Lecithin wird in 12 Teilen Propylenglycol eingebracht. Die entstandene Lösung wird in die Voremulsion eingerührt und homogenisiert. Nach Zugabe von 1 Teil Polyacrylsäure werden 15 Teile Azelainsäure in die entstandene pharmazeutischen Zusammensetzung eingebracht. Danach erfolgt die Ausrichtung des Gelbildners mit der notwendigen Menge an Natriumhydroxid - Lösung. Das resultierende Gel besitzt eine etwa 4-fach höhere Verfügbarkeit von Azelainsäure in lebenden Hautschichten und / oder Hautanhangsorganen.

### 2. Nachweis der Bioverfügbarkeit in der Haut

Die perkutane Resorption von Azelainsäure wurde aus erfindungsgemäßen Hydrogel-Formulierungen im Vergleich zu Skinoren®-Creme untersucht. Das Ziel der Studie war, die Bioverfügbarkeit von Azelainsäure in der Haut. die Penetration von Azelainsäure durch die Haut sowie den dermalen Metabolismus von Azelainsäure zu beschreiben. Dazu wurden verschiedene ¹⁴C-markierte Azelainsäure enthaltende Zubereitungen im in vitro- Modell der FRANZ-Diffusionskammer auf intakte Vollhaut von haarlosen Mäusen (MF1 hr/hr Ola/Hsd; Züchter: Winkelmann, Deutschland) aufgetragen. Eine Menge von 10 bis 15 mg der folgenden Zusammensetzungen wurde auf ein Hautareal von 2 cm² appliziert:
A) 20 % Azelainsäure in einer Creme, deren Hilfsstoff - Zusammensetzung derjenigen von Skinoren® - Creme entspricht.
B) 15 % Azelainsäure in einer erfindungsgemäßen Zusammensetzung.

Der Zeitverlauf der ¹⁴C- Azelainsäure wurde im Akzeptor - Medium (Hepes Hanks Balanced Salt Solution: HHBSS), das unter der Haut entlang strömt, in zweistündigen Intervallen über einen Zeitraum von 24 Stunden gemessen. Weiterhin wurde am Ende eine jeden Experiments die Radioaktivität auf der Hautoberfläche, im Stratum corneum sowie in der restlichen Haut bestimmt. Um den Metabolismus von Azelainsäure in der Haut zu untersuchen, wurden Hautextrakte und ausgewählte Fraktionen von Akzeptor - Medium mittels Radiochromatographie (HPLC und radiometrischer Detektion) untersucht.

Die am Ende des Experimentes in der Haut gefundene ¹⁴C- Azelainsäure wurde nur zu einem Anteil von 3 bis 11 % metabolisiert. Daher sind die Angaben über die Radioaktivität in der Haut praktisch mit der Konzentration unveränderter Azelainsäure gleichzusetzen.

Die folgende Tabelle enthält eine Zusammenfassung der Ergebnisse:

| | **Creme nach dem Stand der Technik** | | **erfindungsgemäße Zusammensetzung** | |
|---|---|---|---|---|
| | Mittelwert | SD | Mittelwert | SD |
| Nicht eingedrungene Azelainsäure | 79,9 % | 14,3 % | 63.6 % | 20,6 % |
| Azelainsäure in dem Stratum corneum | 0,9 % | 0,7 % | 4,0 % | 1,9 % |
| Azelainsäure in der übrigen Haut | 3,7 % | 2,0 % | 27,3 % | 17,1 % |
| Azelainsäure im Akzeptor Medium | 16,3 % | 6,3 % | 5,8 % | 3,3 % |
| SD = Standardabweichung | | | | |

Im Vergleich zur Standard - Creme wurden mit dem erfindungsgemäßen Mittel deutlich höhere Azelainsäure - Konzentrationen in der Haut erzielt.

## Patentansprüche

1. Zusammensetzung, die folgenden Merkmale umfassend:
(i) Azelainsäure als therapeutischer Wirkstoff
(iii) Triacylglyceride,
(iv) Propylenglykol, und
(v) Polysorbate
(vii) in einer wässrigen Phase, umfassend Wasser und Salze,
**dadurch gekennzeichnet,**
**dass** die Zusammensetzung als weitere Zusätze
(ii) Polyacrylsäure und
(vi) Lecithin umfasst,
wobei das Lecithin in höchstens 3 Gewichtsprozent in der Zusammensetzung vorliegt und die Zusammensetzung ein Hydrogel ist.

2. Zusammensetzung nach Anspruch 1, **dadurch gekennzeichnet, dass** die Zusammensetzung topisch verabreichbar ist.

3. Zusammensetzung nach einem der vorherigen Ansprüche, **dadurch gekennzeichnet, dass** die Azelainsäure in einer Konzentration von 5 bis 20 Gewichtsprozent vorliegt.

4. Zusammensetzung nach einem der vorherigen Ansprüche, **dadurch gekennzeichnet, dass** das Lecithin Sojalecithin ist.

5. Zusammensetzung nach einem der vorherigen Ansprüche, **dadurch gekennzeichnet, dass** das Lecithin in einer Konzentration von höchstens 1 Gewichtsprozent vorliegt.

6. Zusammensetzung nach einem der Ansprüche 1 bis 5, **dadurch gekennzeichnet, dass** die Polyacrylsäure in einer Konzentration von 0,5 bis 2 Gewichtsprozenten in der Zusammensetzung vorliegt

7. Zusammensetzung nach Anspruch 6, **dadurch gekennzeichnet, dass** die Polyacrylsäure in einer Konzentration von 1 ± 0,25 Gewichtsprozenten in der Zusammensetzung vorliegt.

8. Zusammensetzung nach einem der Ansprüche 1 bis 5, **dadurch gekennzeichnet, dass** die Triacylglyceride in einer Konzentration von 0,5 bis 5 Gewichtsprozenten in der Zusammensetzung vorliegen.

9. Zusammensetzung nach Anspruch 8, **dadurch gekennzeichnet, dass** die Triacylglyceride in einer Konzentration von 2 ± 1 Gewichtsprozenten in der Zusammensetzung vorliegen.

10. Zusammensetzung nach einem der Ansprüche 1 bis 5, **dadurch gekennzeichnet, dass** das Propylenglykol in einer Konzentration von 5 bis 15 Gewichtsprozenten in der Zusammensetzung vorliegt.

11. Zusammensetzung nach Anspruch 10, **dadurch gekennzeichnet, dass** das Propylenglykol in einer Konzentration von 10 ± 2 Gewichtsprozenten in der Zusammensetzung vorliegt.

12. Zusammensetzung nach einem der Ansprüche 1 bis 5, **dadurch gekennzeichnet, dass** die Polysorbate in einer Konzentration von 0,5 bis 3 Gewichtsprozenten in der Zusammensetzung vorliegen.

13. Zusammensetzung nach Anspruch 12, **dadurch gekennzeichnet, dass** die Polysorbate in einer Konzentration von 2 ± 0,5 Gewichtsprozenten in der Zusammensetzung vorliegen.

14. Zusammensetzung nach einem der vorherigen Ansprüche zur Anwendung als Arzneimittel.

15. Zusammensetzung nach Anspruch 14, **dadurch gekennzeichnet, dass** sie mindestens einen physiologisch verträglichen, pharmakologischen Hilfsstoff oder Trägerstoff umfasst.

16. Zusammensetzung nach Anspruch 14 oder 15 zur Anwendung bei der Behandlung von Rosacea, Altershaut, Melasma, Akne und/oder Hautirritationen.

## Claims

1. Composition that comprises the following features:
(i) Azelaic acid as a therapeutic active ingredient,
(iii) triacylglycerides,
(iv) propylene glycol, and
(v) polysorbates
(vii) in an aqueous phase that comprises water and salts,
**characterized in that**
the composition comprises
(ii) polyacrylic acid, and
(vi) lecithin
as further additives, wherein the lecithin is present in the composition at a concentration of up to 3 % by weight,
and
that the composition is a hydrogel.

2. Composition according to claim 1, **characterized in that** the composition can be administered topically.

3. Composition according to any one of the preceding claims, **characterized in that** the azelaic acid is present at a concentration of 5 to 20% by weight.

4. Composition according to any one of the preceding claims, **characterized in that** the lecithin is soybean lecithin.

5. Composition according to any one of the preceding claims, **characterized in that** the lecithin is present at a concentration of up to 1% by weight.

6. Composition according to any one of claims 1 to 5, **characterized in that** the polyacrylic acid is present in the composition at a concentration of 0.5 to 2% by weight.

7. Composition according to claim 6, **characterized in that** the polyacrylic acid is present in the composition at a concentration of 1 ± 0.25% by weight.

8. Composition according to any one of claims 1 to 5, **characterized in that** the triacylglycerides are present in the composition at a concentration of 0.5 to 5% by weight.

9. Composition according to claim 8, **characterized in that** the triacylglycerides are present in the composition at a concentration of 2 ± 1% by weight.

10. Composition according to any one of claims 1 to 5, **characterized in that** the propylene glycol is present in the composition at a concentration of 5 to 15% by weight.

11. Composition according to claim 10, **characterized in that** the propylene glycol is present in the composition at a concentration of 10 ± 2% by weight.

12. Composition according to any one of claims 1 to 5, **characterized in that** the polysorbates are present in the composition at a concentration of 0.5 to 3% by weight.

13. Composition according to claim 12, **characterized in that** the polysorbates are present in the composition at a concentration of 2 ± 0.5% by weight.

14. Composition according to any one of the preceding claims for use as a medicament.

15. Composition according to claim 14, **characterized in that** it comprises at least one physiologically acceptable pharmacological adjuvant or carrier.

16. Composition according to claim 14 or 15 for use in treating rosacea, presbyderma, melasma, acne, and/or skin irritations.

## Revendications

1. Composition comprenant les caractéristiques suivantes :
(i) l'acide azélaïque en tant que substance active thérapeutique,
(iii) des triacylglycérides,
(iv) le propylèneglycol, et
(v) des polysorbates,
(vii) dans une phase aqueuse comprenant de l'eau et des sels,
**caractérisée en ce que** la composition comprend, en tant qu'additifs supplémentaires
(ii) de l'acide polyacrylique et
(vi) de la lécithine,
la lécithine étant présente dans la composition à au plus 3 pour cent en poids et la composition étant un hydrogel.

2. Composition selon la revendication 1, **caractérisée en ce que** la composition est administrable par voie topique.

3. Composition selon l'une quelconque des revendications précédentes, **caractérisée en ce que** l'acide azélaïque est présent en une concentration de 5 à 20 pour cent en poids.

4. Composition selon l'une quelconque des revendications précédentes, **caractérisée en ce que** la lécithine est la lécithine de soja.

5. Composition selon l'une quelconque des revendications précédentes, **caractérisée en ce que** la lécithine est présente en une concentration d'au plus 1 pour cent en poids.

6. Composition selon l'une quelconque des revendications 1 à 5, **caractérisée en ce que** l'acide polyacrylique est présent en une concentration de 0,5 à 2 pour cent en poids dans la composition.

7. Composition selon la revendication 6, **caractérisée en ce que** l'acide polyacrylique est présent en une concentration de 1 ± 0,25 pour cent en poids dans la composition.

8. Composition selon l'une quelconque des revendications 1 à 5, **caractérisée en ce que** les triacylglycérides sont présents en une concentration de 0,5 à 5 pour cent en poids dans la composition.

9. Composition selon la revendication 8, **caractérisée en ce que** les triacylglycérides sont présents en une concentration de 2 ± 1 pour cent en poids dans la composition.

10. Composition selon l'une quelconque des revendications 1 à 5, **caractérisée en ce que** le propylèneglycol est présent en une concentration de 5 à 15 pour cent en poids dans la composition.

11. Composition selon la revendication 10, **caractérisée en ce que** le propylèneglycol est présent en une concentration de 10 ± 2 pour cent en poids dans la composition.

12. Composition selon l'une quelconque des revendications 1 à 5, **caractérisée en ce que** les polysorbates sont présents en une concentration de 0,5 à 3 pour cent en poids dans la composition.

13. Composition selon la revendication 12, **caractérisée en ce que** les polysorbates sont présents en une concentration de 2 ± 0,5 pour cent en poids dans la composition.

14. Composition selon l'une quelconque des revendications précédentes pour une utilisation en tant que médicament.

15. Composition selon la revendication 14, **caractérisée en ce qu'**elle comprend au moins un auxiliaire ou substance de support pharmacologique physiologiquement acceptable.

16. Composition selon la revendication 14 ou 15 pour une utilisation lors du traitement de la rosacée, de taches de vieillesse, du mélasme, de l'acné et/ou d'irritations cutanées.
